# EUROPÄISCHE PATENTSCHRIFT

(11) **EP 2 237 763 B1**
(45) Veröffentlichungstag und Bekanntmachung des Hinweises auf die Patenterteilung: **15.05.2013**
(21) Anmeldenummer: 08867525.1
(22) Anmeldetag: 18.12.2008
(51) Int. Cl.: A61K 6/083

(54) **STUMPFAUFBAUMATERIAL**
MATERIAL FOR BUILDING UP STUMPS
MATÉRIAU DE RECONSTITUTION DE MOIGNON

(30) Priorität: 21.12.2007 EP 07024908
(43) Veröffentlichungstag der Anmeldung: 13.10.2010
(73) Patentinhaber: Ernst Mühlbauer GmbH & Co.KG, 25870 Norderfriedrichskoog (DE)
(72) Erfinder: NEFFGEN, Stephan, 25421 Pinneberg (DE); HAUSER, Karsten, 22529 Hamburg (DE)
(74) Vertreter: Glawe, Delfs, Moll
(86) Internationale Anmeldenummer: PCT/EP2008/010843
(87) Internationale Veröffentlichungsnummer: WO 2009/083168

(56) Entgegenhaltungen:
- EP-A- 0 530 926
- EP-A- 1 570 831
- WO-A-99/65453
- US-A1- 2005 252 413

## Beschreibung

Die Erfindung betrifft ein polymerisierbares Material zur Herstellung eines dentalen Stumpfaufbaumaterials.

Ein Stumpfaufbaumaterial dient zum Aufbau eines stark zerstörten Zahnes, so dass der aufgebaute Zahn sicher eine Krone oder Brücke halten kann. Nach dem Aufbringen des Stumpfaufbaumaterials auf den lädierten Zahn wird das Material modelliert und ausgehärtet. Anschließend wird der wiederaufgebaute Zahn so geschliffen, dass ein zum Tragen einer Krone oder eines Brückenglieds geeigneter Zahnstumpf entsteht. Stumpfaufbaumaterialien sind bspw. offenbart in EP-A-1 790 323.

EP 1 570 831 A1 offenbart ein gefülltes, polymerisierbares Dentalmaterial. Es werden teilweise agglomerierte Nanofüllstoffe verwendet. WO 99/65453 A1 offenbart ein Hybridkomposit, bei dem Füllstoffe bis auf kleine Partikelgrößen gemahlen werden. US 2005/0252413 A1 offenbart Glasionomerzement, der Nanofüllstoff enthält. EP 0 530 926 A1 beschreibt einen Dentalfüllstoff, der eine Kombination aus Mikro- und Nanofüllstoffen enthält.

Der Erfindung liegt die Aufgabe zugrunde, ein polymerisiebares Material zur Herstellung eines dentalen Stumpfaufbaumaterials zu schaffen, das gut verarbeitbar ist und das nach seiner Aushärtung zum Stumpfaufbaumaterial eine gute Weiterbearbeitung erlaubt.

Erfindungsgemäß weist das polymerisierbare Material dazu folgende Merkmale auf:
- das Material enthält wenigstens ein polymerisierbares Monomer, Oligomer und/oder Präpolymer,
- der Gesamtfüllstoffgehalt des Materials beträgt 50 bis 80 Gew.-%,
- das Material enthält 6 bis 18 Gew.-% Nanofüllstoff mit einer mittleren Teilchengröße (bestimmt mittels dynamischer Lichtstreuung (3D-PCS) an verdünnten Dispersionen in 2-Butanon mit einem Feststoffanteil von etwa 0,5 Gew.-%) d₅₀ von 300 nm oder weniger, wobei wenigstens 50 Gew.-% dieses Nanofüllstoffs eine Teilchengröße von 200 nm oder weniger aufweist,
- das Material enthält 35 bis 60 Gew.-% Mikrofüllstoff mit einer mittleren Teilchengröße (bestimmt mittels Laserdiffraktometrie) d₅₀ von 0,5 bis 10 µm,
- die Beschleifbarkeit des ausgehärteten Stumpfaufbaumaterials weicht höchstens 15 % von der Beschleifbarkeit von Humandentin ab, wobei diese Beschleifbarkeit wie folgt ermittelt wird:
- es wird ein zylindrischer Diamantschleifkörpers mit einer Körnung von 100 µm (Typ ISO 836.314.012) bei 150.000 U/min verwendet,
- der Prüfkörper wird mit einem Zuggewicht von 100 g an dem Diamantschleifkörper vorbeigeführt und beschliffen,
- gemäß ISO-Norm 11405 wird der Schleifkörper mit Spraywasser bei einer Durchflussgeschwindigkeit von 50 ml/min und einer Temperatur von 23° C gekühlt ,
zur Verwendung als dentales Stumpfaufbaumaterial.

Kern der Erfindung ist das Einbringen einer Füllstoffkombination in das Material, die zu einer Beschleifbarkeit des ausgehärteten Stumpfaufbaumaterials führt, die der Beschleifbarkeit von Humandentin angenähert ist.

Zur Ermittlung der Beschleifbarkeit werden zunächst Prüfkörper aus ausgehärtetem Stumpfaufbaumaterial hergestellt. In der nachfolgenden Erläuterung der Herstellung der Prüfkörper und des Messverfahrens zur Ermittlung der Beschleifbarkeit wird davon ausgegangen, dass ein lichthärtendes Material verwendet wird. Das nachfolgend beschriebene Verfahren lehnt sich an ein in der Literatur beschriebenes Verfahren (Dissertation von Iwer Lasson, Titel "Mechanische Eigenschaften verschiedener Aufbaumaterialien", Universitätsklinikum Hamburg-Eppendorf, 2005).

Prüfkörper der Stumpfaufbaumaterialien werden in einer Kunststoffform mit einer Länge von 40mm, einer Breite von 10mm und einer Höhe von 5mm hergestellt. Die Form wird auf einen Objektträger gelegt. Nach dem Einbringen des polymerisierbaren Materials wird die Form mit Folie bedeckt und ein zweiter Objektträger auf das Material gedrückt, so dass der Überschuss verdrängt wird und entfernt werden kann. Die Objektträger werden mit einer Federklammer fixiert. Danach wird der Prüfkörper von beiden Seiten je 90s in einem Lichtofen (Typ HeraFlash der Firma Kulzer) belichtet. Danach werden sie aus der Form entnommen und 16h in destilliertem Wasser bei 37°C gelagert. Von jedem Material werden mindestens 4 Prüfkörper hergestellt.

Als Referenz dient humanes Dentin. Dazu werden die Kronen von extrahierten menschlichen Molaren mit einer Diamantsäge zu Rechtecken beschliffen, wodurch der Schmelz entfernt wird. Im Anschluss werden die Kronen mit der Diamantsäge zu Dentinblöcken mit einer Breite von 10mm und einer Höhe von 5mm verarbeitet (s. auch S. 38-39 der Dissertation). Dann werden die Dentinblöcke mit einem Stumpfaufbaumaterial so eingebettet, dass das Humandentin beschliffen werden kann. Die Aushärtung des Stumpfaufbaumaterials erfolgt wie oben beschrieben.

Bis zur Messung werden alle Prüfkörper bei Raumtemperatur in destilliertem Wasser gelagert.

Die Schleifversuche werden durchgeführt auf einem druckluftgelagerten Schwebetisch (Luftlagerschlitten) mit Haltevorrichtung für den Prüfkörper, einer Halterung für ein Winkelstück mit Schleifkörper und einer Regeleinheit für die Umdrehungszahl, Spraywasser und Sprayluft. Es wird ein zylindrischer Diamantschleifkörper mit einer Körnung von 100µm (Typ ISO 836.314.012) der Firma Brasseler verwendet. Als Halterung für die Schleifkörper dient ein handelsübliches Winkelstück mit Mikromotor der Firma KaVo.

Bei der Durchführung der Beschleifbarkeitsmessung werden die Prüfkörper mit Hilfe der Haltevorrichtung auf dem Luftlagerschlitten montiert. Zur Feinjustierung wird der Prüfkörper mit Hilfe einer Mikrometerschraube um einen Betrag von 0,2mm an den Schleifkörper herangeführt. Dann wird der auf dem Luftlagerschlitten befestigte Prüfkörper durch ein Zuggewicht von 100g an dem starr montierten Winkelstück mit Schleifkörper vorbeigeführt und dabei beschliffen. Die Drehzahl des Winkelstücks wird auf 150.000 U/min eingestellt und um der ISO-Norm 11405 zu entsprechen, wird der Schleifkörper mit Spraywasser bei einer Durchflussgeschwindigkeit von 50ml/min und einer Temperatur von 23°C gekühlt. Zur Bestimmung der Beschleifbarkeit wird per Stoppuhr die Zeit gemessen, die der Schleifkörper für das Zurücklegen einer Strecke von 10mm auf der Prüfkörperoberfläche benötigt. Nach jeweils 30 Messungen wird ein neuer Schleifkörper verwendet.

Das geschilderte Vorgehen entspricht der Beschreibung in den Seiten 42 bis 44 der Dissertation. Einzige Abweichung ist die Verwendung eines Zuggewichts von 100g, da dies die Reproduzierbarkeit der Ergebnisse erhöht.

Eine Abweichung der Beschleifbarkeit des ausgehärteten erfindungsgemäßen Materials von höchstens 15%, vorzugsweise höchstens 10% verglichen zu der Beschleifbarkeit von Humandentin bedeutet somit im Sinne des Patentanspruchs, dass die gemessene Zeit, in der der Schleifkörper eine Strecke von 10mm auf der Prüfkörperoberfläche zurücklegt, höchstens 15% bzw. 10% von der entsprechenden zeit beim Beschleifen von Humandentin abweicht.

Der Harzanteil des erfindungsgemäßen Materials enthält polymerisierbare Monomere, Oligomere und/oder Präpolymere, die bevorzugt ausgewählt sind aus der Gruppe bestehend aus radikalisch und kationisch polymerisierbaren Monomeren, Oligomeren und/oder Präpolymeren. Radikalisch polymerisierbare Monomere, Oligomere und/oder Präpolymere, insbesondere radikalisch polymerisierbare Monomere, sind bevorzugt. Die Initiierung der radikalischen Polymerisation kann durch geeignete Initiatoren, insbesondere Lichthärter und/oder chemische Startersubstanzen, erfolgen. Chemische Initiatorsysteme können insbesondere dann verwendet werden, wenn das polymerisierbare Material als zwei- oder Mehrkomponentensystem gelagert und erst unmittelbar vor der Verwendung angemischt wird.

Als geeignete Monomere werden besonders bevorzugt Acrylate, Methacrylate, Acrylamide, Methacrylamide, Vinylether, Epoxide, Oxetane, Spiroorthocarbonate, Spiroorthoester, bizyklische Orthoester, bizyklische Monolactone, bizyklische Bislactone, zyklische Carbonate, zyklische Acetale, Allylsulfide, Vinylzyklopropane, organische Phosphate, organische Phosponate, organische Phosphite oder eine Kombination aus diesen Verbindungen eingesetzt. Die Allgemeinheit nicht einschränkend sind nachfolgend einige Beispiele genannt: Methyl(meth)acrylat, Ethyl(meth)acrylat, n- oder i-Propyl(meth)acrylat, n-, i- oder tert.-Butyl(meth)acrylat, Hexyl(meth)acrylat, 2-Ethylhexyl(meth)acrylat, Cyclohexyl(meth)acrylat, Isobornyl(meth)acrylat, Hydroxyethyl(meth)acrylat, Hydroxypropyl(meth)acrylat, Phosphorsäureester von Hydroxyethyl(meth)acrylat bzw. Hydroxypropyl(meth)acrylat, (Meth)acrylsäure, Malonsäure-mono-(meth)acrylatester, Bersteinsäure-mono-(meth)acrylatester, Maleinsäure-mono-(meth)acrylatester, Glycerin(meth)acrylat, Glycerin(meth)acrylatester, Glycerindi(meth)acrylat, Glycerin-di(meth)acrylatester (wie z. B. Glycerin-di(meth)acrylatsuccinat), 4-(Meth)acryloyloxyethyltrimellithsäure, Bis-4,6- bzw. Bis-2,5-(Meth)acryloyloxyethyltrimellithsäure, 2-(((Alkylamino)-carbonyl)oxy)ethyl-(meth)acrylate, Allyl(meth)acrylat, Butandioldi(meth)acrylat, Hexandiol-di(meth)acrylat, Decandioldi(meth)acrylat, Dodecandiol-di(meth)acrylat, Ethylenglykoldi(meth)acrylat, Diethylenglykoldi(meth)acrylat, Triethylenglykoldi(meth)acrylat, Tetraethylenglykoldi(meth)acrylat, Polyethylenglykol-di(meth)acrylate, Glycerindi(meth)acrylat, Glycerolpropoxytri(meth)acrylat, Trimethylolpropantri(meth)acrylat, ethoxylierte und/oder propoxylierte Trimethylolpropantri(meth)acrylate, Pentaerythrittetra(meth)acrylat, Di-pentaerythritolhexa(meth)acrylat, Bisphenol-A-di(meth)acrylat, ethoxylierte und/oder propoxylierte Bisphenol-A-di(meth)acrylate, 2,2-Bis-4-(3-(meth)acryloxy-2-hydroxy-propoxy)-phenylpropan und davon abgeleitete Verbindungen, Chlor- und Bromphosphorsäureester des Bisphenol-A-glycidyl(meth)acrylats, Urethan(meth)acrylate (wie z. B. 7,7,9-Trimethyl-4,13-dioxo-3,14-dioxa-5,12-diazahexadecan-1,16-dioxy-dimethacrylat), Polyesterurethan(meth)acrylate, Polyester(meth)acrylate, Polycarbonat(meth)acrylate, Polyamid(meth)acrylate, Polyimid(meth)acrylate, Phosphazen(meth)acrylate und Siloxan(meth)acrylate; Ethylvinylether, n- oder i-Propylvinylether, n-, i- oder tert.-Butylvinylether, Hexylvinylether, Octylvinylether, Cyclohexylvinylether, Cyclohexyl-3,4-epoxy-1-methylvinylether, Dimethanol-cyclohexyl-monovinylether, 1,4-Dimethanolcyclohexyl-divinylether, Propandioldivinylether, Butandioldivinylether, Hexandioldivinylether, Octandioldivinylether, Decandiolvinylether, Ethylenglykoldivinylether, Diethylenglykoldivinylether, Triethylenglykoldivinylether, Triethylenglykol-monovinylether-mono(meth)acrylsäureester, Polyethylenglykoldivinylether, Tripropylenglykoldivinylether, Glycerintrivinylether, Pentaerythrittetravinylether,
7,7,9-Trimethyl-4,13-dioxo-3,14-dioxa-5,12-diazahexadecan-1,16-dioxydivinylether, Bisphenol-A-divinylether, ethoxylierte und/oder propoxylierte Bisphenol-A-divinylether , Polyestervinylether, Polycarbonatvinylether, Polyacrylatvinylether, Polyamidvinylether, Polyimidvinylether, Polyurethanvinylether, Phosphazenvinylether und Siloxanvinylether; Alkylglycidylether, Glycidol, Glycidyl(meth)acrylat, Dipentendioxid, 1,2-Epoxyhexadecan, Bis-(3,4-epoxycyclohexyl)-adipat, Vinylcyclohexenoxide, vinylcyclohexendioxide, Epoxycyclohexancarboxylate (wie z. B. 3,4-Epoxycyclohexylmethyl-3,4-epoxycyclohexen-carboxylat), Butandioldiglycidylether, Hexandioldiglycidylether, Dodecandioldiglycidylether, Diglycidylether der Polyethylenglykole und der Polypropylenglykole, Diglycidylether von substituierten (z. B. halogenierten) und unsubstituierten Bisphenolen (wie z. B. Bisphenol-A, Bisphenol-C und Bisphenol-F), Resorcindiglycidylether, Trimetylolethantriglycidylether, Trimetylolpropantriglycidylether, Polybutadienpolyepoxide, Polyesterepoxide, Polycarbonatepoxide, Polyacrylatepoxide, Polyamidepoxide, Polyimidepoxide, Polyurethanepoxide, Phosphazenepoxide und Siloxanepoxide; 3,3-disubstituierte Oxetane und Dioxetane (wie z.B. 3-Ethyl-3-(2-hydroxyethyl)-oxetan); (trans/trans)-2,3,8,9-Di(tetramethylen)-1,5,7,11-tetraoxaspira-[5.5]-undecan; substituierte 1,3-Dioxolane (wie z. B. 2-Phenyl-4-methylen-1,3-dioxolan); difunktionelle 6-Methylen-1,4-dithiepane sowie die Umsetzungsprodukte von nukleophilen (Meth)acrylaten wie z. B. 2-Hydroxyethyl(meth)acrylat oder Glycerin(meth)acrylatestern mit reaktiven Phosphorsäure-, Phosphonsäure- oder Phosphinsäurederivaten wie z. B. P₂O₅, POCl₃ oder PCl₃.

Acrylate und/oder Methacrylate sind besonders bevorzugte polymerisierbare Monomere. Besonders bevorzugt sind Bisphenol-A-glycidylmethacrylat (Bis-GMA), Triethylenglykoldimethacrylat (TEDMA), Urethandimethacrylat (UDMA), Dodecandimethacrylat, Hexandioldimethacrylat und vergleichbare, für Dentalmaterialien übliche Acrylate bzw. Methacrylate.

Im Rahmen der Erfindung sind die Untergrenzen für den Gesamtfüllstoffgehalt 50, 55, 58 und 60 Gew.-%. Die Obergrenzen sind 80, 75, 72 und 70 Gew.-%. Im Rahmen der Erfindung sind diese Ober- und Untergrenzen beliebig zu Bereichen kombinierbar.

Untergrenzen für den Gehalt an Nanofüllstoff sind 6 und 8 Gew.-%. Obergrenzen sind 18 Gew.-%. Die genannten Ober- und Untergrenzen lassen sich beliebig zu erfindungsgemäßen Bereichen kombinieren.

Die mittlere Teilchengröße d₅₀ des erfindungsgemäß verwendeten Nanofüllstoffs liegt bei 300 nm oder weniger, wobei wenigstens 50 Gew.-% dieses Nanofüllstoffs eine Teilchengröße von 200 nm oder weniger aufweist.

Die erfindungsgemäß verwendeten Nanopartikel können als isolierte Partikel oder auch agglomerierte und/oder aggregierte Partikel zum Einsatz kommen. Bei isolierten Nanopartikel handelt es sich um diskrete Gebilde, die durch z.B. elektronenmikroskopische Methoden keine weiteren Untereinheiten erkennen lassen. Der Begriff "aggregiert" beschreibt stark assoziierte Gebilde, bei denen die elektronenmikroskopisch erkennbaren Untereinheiten durch sehr starke Kräfte, bis hin zu Sinterbrücken, zusammengehalten werden. Aggregierte Partikel entstehen in der Regel aus zunächst isolierten Partikeln in sekundären Prozessen, wobei die Partikel in Folge der Sekundärprozesse die späteren Untereinheiten bilden. Der Begriff agglomeriert beschreibt die schwache Assoziation von Partikeln, bspw. aufgrund von polaren Wechselwirkungen zwischen den das Agglomerat aufbauenden Partikeln. Auch hier sind die individuellen Partikel des Agglomerats elektronenmikroskopisch erkennbar.

Die Aggregate und Agglomerate aufbauenden Untereinheiten (Partikel) im Sinne dieser Erfindung werden als Primärpartikel oder Primärteilchen bezeichnet. Der Durchmesser der Primärpartikel lässt sich bevorzugt durch Transmissionselektronenmikroskopie ermitteln und wird als Primärpartikelgröße bezeichnet. Durch statistische Auswertung der elektronenmikroskopischen Aufnahmen von vielen assoziierten Primärpartikeln lassen sich Mittelwerte und andere statistische Größen bestimmen.

Werden die erfindungsgemäß eingesetzten Nanopartikel in einer flüssigen Phase dispergiert, so können sie je nach eingesetzter Form, Dispergierverfahren und Oberflächeneigenschaften der Partikel in isolierter Form oder aber als Agglomerate und/oder Aggregate vorliegen. Die Durchmesser der Gebilde (isolierte Partikel, Agglomerate, Aggregate) in dispergierter Form werden als Teilchengröße bezeichnet. Die Ermittelung der Teilchengröße kann durch die Untersuchung der Dispersionen mithilfe der Photokorrelationsspektroskopie (auch dynamische Lichtstreuung genannt) oder auch modifizierter Formen der Fraunhofer Beugung, wie bspw. der sog. PIDS-Technologie (z.B. Fa. Beckman Coulter, Europark Fichtenhain B13, 47807 Krefeld, Deutschland), vorgenommen werden.

Die mittlere Primärteilchengröße des erfindungsgemäßen nanoskaligen Füllstoffs liegt bevorzugt zwischen 1 nm und 80 nm, weiter bevorzugt zwischen 4 nm und 60 nm und besonders bevorzugt zwischen 6 nm und 50 nm. Der erfindungsgemäße Nanofüllstoff weist bevorzugt eine BET-Oberfläche (gemäß DIN 66131 bzw. DIN ISO 9277) zwischen 15 m²/g und 600 m²/g, bevorzugt zwischen 30 m²/g und 500 m²/g und besonders bevorzugt zwischen 50 m²/g und 400 m²/g auf.

Bei den erfindungsgemäß eingesetzten Nanofüllstoffen handelt es sich bevorzugt um Metall-, Halbmetall- oder Mischmetalloxide, -Silikate, -Nitride, -Sulfate, -Titanate, -Zirkonate, -Stannate, -Wolframate oder um eine Mischung aus diesen Verbindungen. Zur Gruppe der Halbmetalle, deren Eigenschaften (vor allem Aussehen und elektrische Leitfähigkeit) zwischen denen der Metalle und der Nichtmetalle liegen, gehören Bor, Silizium, Germanium, Arsen, Antimon, Bismut, Selen, Tellur und Polonium (vgl. Römpp Chemie Lexikon, Georg Thieme Verlag, 1990, S. 1711). Die Gruppe der Metalle ist im Periodensystem links von der Gruppe der Halbmetalle zu finden, d.h. dazu gehören die Hauptgruppenmetalle, Nebengruppenmetalle, Lanthanide und Actinide. Und unter dem Begriff Mischmetalloxid, -nitrid, usw. ist hier eine chemische Verbindung zu verstehen, in der mindestens zwei Metalle und/oder Halbmetalle zusammen mit dem entsprechenden Nichtmetallanion (Oxid, Nitrid, usw.) chemisch miteinander verbunden sind.

Bei den erfindungsgemäß eingesetzten Nanofüllstoffen handelt es sich besonders bevorzugt um Siliziumdioxid, Aluminiumoxid, Zirkondioxid, Titandioxid, Zinkoxid, Zinndioxid, Ceroxid, Aluminium-Silizium-Oxide, Silizium-Zink-Oxide, Silizium-Zirkon-Oxide, Eisenoxide und deren Mischungen mit Siliziumdioxid, Indiumoxide und deren Mischungen mit Siliziumdioxid und/oder Zinndioxid, Bornitrid, Strontiumsulfat, Bariumsulfat, Strontiumtitanat, Bariumtitanat, Natriumzirkonat, Kaliumzirkonat, Magnesiumzirkonat, Calciumzirkonat, Strontiumzirkonat, Bariumzirkonat, Natriumwolframat, Kaliumwolframat, Magnesiumwolframat, Calciumwolframat, Strontiumwolframat und/oder Bariumwolframat. Mischoxide der vorgenannten Stoffe sind ebenfalls bevorzugt.

Als Nanofüllstoff bevorzugt sind aus pyrogenen Kieselsäuren (erhältlich beispielsweise von der Firma Degussa unter dem Handelsnamen Aerosil®) herstellbare Partikel gemäß der Offenbarung der WO 2005/084611 A1. Ebenfalls bevorzugt sind aus Kieselsäuresolen herstellbare Nanopartikel, deren Oberfläche organisch modifiziert ist, wie bspw. offenbart in US-A-6 899 948, EP 1 236 765 A1 und EP 803 240 A1.

Im Rahmen der Erfindung ist die Untergrenze für den Gehalt an Mikrofüllstoff 35 Gew.-%. Die Obergrenze ist 60 Gew.-%. Diese Ober- und Untergrenzen sind beliebig zu erfindungsgemäßen Bereichen kombinierbar.

Die mittlere Teilchengröße (d₅₀) des Mikrofüllstoffs beträgt wenigstens 0,5, 0,7 und 1,0 µm. Obergrenzen für die mittlere Teilchengröße des Mikrofüllstoffs sind 10,0, 7,0, 5,0, 4,0 und 3,0 µm. Die Ober- und Untergrenzen sind beliebig zu erfindungsgemäßen Bereichen kombinierbar.

Der Mikrofüllstoff kann ein nichtreaktiver Füllstoff, ein reaktiver Füllstoff oder eine Mischung aus diesen beiden Füllstoff-Typen sein. Unter einem reaktiven Füllstoff wird hier ein Füllstoff verstanden, der unter Zutritt von Wasser Ionen freisetzt und somit zu einer Aushärtung des Materials über eine Säure-Base-Reaktion führen kann. Diese reaktiven Füllstoffe werden z.B. zur Herstellung von Compomeren und Glasionomerzementen verwendet und sind z.B. in D.C. Smith, Biomaterials 19, S. 467-478 (1998) beschrieben.

Die Messung der Teilchengröße von Nano- und Mikrofüllstoffen kann auf unterschiedliche Art und Weise erfolgen. Die bekannten Methoden führen sämtlich zu absoluten und damit untereinander vergleichbaren Bestimmungen der Teilchengröße. Beispielhaft genannt seien statische oder dynamische Lichtstreuung, Sedimentation im Gravitations- oder Zentrifugalfeld (beispielsweise Ultrazentrifugation), Partikelzählung im Elektrolythen (Coulter-Counter-Methode), Siebung, Bildanalyse oder Videobildanalyse von optischen oder elektronenmikroskopischen Bildern (beispielsweise Transmissionselektronenmikroskopie TEM), Ultraschallspektroskopie und 3D-ORM-Laserrückreflexion. Bevorzugte Methoden zur Teilchengrößebestimmung der Nano- und Mikrofüllstoffe sind weiter unten im Zusammenhang mit den Beispielen genannt.

Als Mikrofüllstoff werden bevorzugt Quarzpulver, Glaspulver, Glaskeramikpulver, Metalloxide, Metallhydroxide, sphärische Füllstoffe wie z.B. in der DE-PS 3247800- beschrieben, amorphe Cluster-Füllstoffe wie z.B. in der WO 01/30306 beschrieben oder eine Mischung aus diesen Füllstoffen eingesetzt.

Als Mikrofüllstoff werden besonders bevorzugt Bariumsilikatgläser, Strontiumsilikatgläser, Borataluminosilikatgläser, Phosphataluminosilikatgläser, Fluoroaluminosilikatgläser, Calciumsilikate, Zirkonsilikate, Natriumaluminiumsilikate, Schichtsilikate, Bentonite, Zeolithe einschließlich der Molekularsiebe, die Oxide sowie die Hydroxide der Alkali- und der Erdalkalimetalle, Apatit, sphärische Füllstoffe wie z.B. in der DE-PS 3247800 beschrieben, amorphe Cluster-Füllstoffe wie z.B. in der WO 01/30306 beschrieben oder eine Mischung aus diesen Füllstoffen eingesetzt.

Eine bevorzugte Variante ist die Verwendung eines Nano-und/oder Mikrofüllstoffs, bei dem durch organische Modifikation funktionelle Gruppen auf die Oberfläche des Füllstoffs aufgebracht werden, die mit dem organischen Bindemittel bzw. organischen Harz chemisch reagieren können oder eine hohe Affinität zu dem organischen Bindemittel haben. Bei diesen funktionellen Gruppen handelt es sich bevorzugt um Acrylat-, Methacrylat-, Cyanacrylat-, Acrylamid-, Methacrylamid-, Vinyl-, Allyl-, Epoxid-, Oxetan-, Vinylether-, Amino-, Säure-, Säureester-, Säurechlorid-, Phosphat-, Phosphonat-, Phosphit-, Thiol-, Alkohol- und/oder Isocyanatgruppen. Bevorzugt werden diese Gruppen über geeignete Verbindungen wie bspw. Siloxane, Chlorsilane, Silazane, Titanate, Zirkonate, Wolframate, organische Säuren, organische Säurechloride oder -anhydride eingeführt.

Die organische Modifikation der Oberfläche der Nanofüllstoffe oder Mikrofüllstoffe erfolgt bevorzugt durch Behandeln mit einem Siloxan, Chlorsilan, Silazan, Titanat, Zirkonat, Wolframat oder mit einer organischen Säure (wie sie z. B. in US 6,387,981 beschrieben sind), einem organischen Säurechlorid oder -anhydrid. Die Siloxane, Chlorsilane, Silazane, Titanate, Zirkonate und Wolframate haben besonders bevorzugt die allgemeinen Formeln Si(OR')ₙR₄₋ₙ, Si ClₙR₄₋ₙ, (RₘR''₃₋ₘSi)₂NH, Ti(OR')ₙR₄₋ₙ, Zr(OR')ₙR₄₋ₙ und W(OR')ₙR₆₋ₙ, wobei m und n 1, 2 oder 3 ist; bevorzugt ist n = 3. Bei der über den Sauerstoff gebundenen Gruppe R' handelt es sich ebenso wie bei R " um eine beliebige organische funktionelle Gruppe, bevorzugt um eine Alkylgruppe und besonders bevorzugt um eine Methyl-, Ethyl-, Propyl- oder Isopropylgruppe. Die funktionelle Gruppe R ist eine beliebige organische Gruppe und direkt über ein Kohlenstoffatom an das Silizium, Titan, Zirkon oder Wolfram gebunden. Wenn m oder n 1 oder 2 ist, können die Gruppen R gleich oder verschieden sein. R wird bevorzugt so ausgewählt, dass es eine oder mehrere funktionelle Gruppen besitzt, die mit dem organischen Harz chemisch reagieren können oder eine hohe Affinität zu dem organischen Harz haben. Diese funktionellen Gruppen sind auch bei den ebenfalls zur organischen Oberflächenmodifikation einsetzbaren, oben aufgeführten organischen Säuren, Säurechloriden und -anhydriden enthalten. Es handelt sich bevorzugt um Acrylat-, Methacrylat-, Cyanacrylat-, Acrylamid-, Methacrylamid-, Vinyl-, Allyl-, Epoxid-, Oxetan-, Vinylether-, Amino-, Säure-, Säureester-, Säurechlorid-, Phosphat-, Phosphonat-, Phosphit-, Thiol-, Alkohol- und/oder Isocyanatgruppen. Die bei der organischen Oberflächenmodifikation der Füllstoffe gebildeten Nebenprodukte wie z. B. Alkohole, Salzsäure oder Ammoniak werden vorzugsweise in den Folgeschritten bis auf mögliche Reste (Verunreinigungen) entfernt, d. h. sie sind in dem später hergestellten Dentalmaterial nicht mehr oder nur in geringen Mengen von ≤ 0,4 Gew.-%, vorzugsweise ≤ 0,2 Gew.-% enthalten.

Mikrofüllstoffe können wie im Stand der Technik bekannt trocken oder in Suspension oberflächenmodifiziert werden. Bezüglich Silanisierung und Silanisierungsverfahren sei auf Ralf Janda, Kunststoffverbundsysteme, 1. Auflage, VCH-Verlagsgesellschaft Weinheim, 1990, S. 93 ff. sowie die dort zitierte Literatur verwiesen.

Bei dem Lösungsmittel, in dem die organische Oberflächenmodifikation der Nanofüllstoffe durchgeführt wird, handelt es sich bevorzugt um ein polar aprotisches Lösungsmittel und besonders bevorzugt um Aceton, Butanon, Essigsäureethylester, Methylisobutylketon, Tetrahydrofuran oder Diisopropylether. Weiterhin ist die direkte organische Oberflächenmodifikation der Nanofüllstoffe in dem zur Herstellung der Dentalmaterialien zu verwendendem organischen Bindemittel eine besonders bevorzugte Verfahrensweise. In diesem Fall ist das organische Harz bzw. Bindemittel das zu verwendende Lösungsmittel. Zur Beschleunigung der organischen Oberflächenmodifikation der Nanofüllstoffe kann eine Säure als Katalysator zugegeben werden. In jedem Fall müssen katalytische Mengen Wasser, bevorzugt zwischen 0,01 % und 5 %, anwesend sein, um die Modifikation durchzuführen. Dieses Wasser ist an den Oberflächen der als Ausgangssubstanz verwendeten Füllstoffe oftmals bereits als Adsorbat vorhanden. Zur Unterstützung der Reaktion kann weiteres Wasser, z. B. auch in Form einer verdünnten Säure zugegeben werden.

Um den Zerfall etwaiger Agglomerate und Aggregate von Nanofüllstoffen bei der organischen Oberflächenmodifikation in dem organischen Lösungsmittel zu beschleunigen, kann vor oder während der Modifizierung ein zusätzlicher Energieeintrag mit den üblichen Methoden erfolgen. Dies kann z. B. durch einen Hochgeschwindigkeitsrührer, einen Dissolver, eine Perlmühle oder einen Mischer erfolgen. Bei der Verwendung höherviskoser Lösungsmittel ist dies die bevorzugte Vorgehensweise, also besonders dann, wenn das organische Bindemittel direkt als Lösungsmittel verwendet wird. Wenn das organische Harz oder Bindemittel nicht als Lösungsmittel verwendet wird, kann das zu verwendende organische Bindemittel direkt mit der Dispersion des organisch modifizierten Nanofüllstoffs in dem organischen Lösungsmittel gefüllt werden. In diesem Fall wird das Lösungsmittel nach der Herstellung der Mischung aus organischem Bindemittel und organisch modifiziertem Nanofüllstoff abgezogen. Bevorzugt wird der organisch modifizierte Nanofüllstoff von dem Lösungsmittel befreit und als trockenes Pulver weiter verarbeitet. In diesem Fall wird dann das trockene organisch modifizierte Nanopulver zu dem organischen Bindemittel gegeben und unter mechanischem Energieeintrag eingearbeitet. Die Einarbeitung kann z. B. durch einen Hochgeschwindigkeitsrührer, einen Dissolver, eine Perlmühle, einen Walzen= stuhl, einen Kneter oder einen Mischer erfolgen.

Bei der Verwendung höherviskoser Lösungsmittel und insbesondere bei der direkten Verwendung des organischen Bindemittels als Lösungsmittel kann es passieren, dass eventuelle Überschüsse und/oder nicht umgesetzte Teile der zur organischen Oberflächenmodifikation der Nanofüllstoffe verwendeten Verbindung nicht aus der Dispersion entfernt werden können. In diesem Fall ist es die bevorzugte Vorgehensweise, dass diese eventuellen Überschüsse und/oder nicht umgesetzten Teile der zur organischen Oberflächenmodifikation der Nanofüllstoffe verwendeten Verbindung durch Reaktion mit einem geeigneten Agens in Stoffe umgesetzt werden, die dann entweder aus der Dispersion entfernt werden oder aber in der Dispersion verbleiben können, wenn sie für den Menschen unbedenklich, d.h. nicht schädigend sind. Eine besonders bevorzugte Vorgehensweise ist die Verwendung von Wasser als Agens, das mit eventuellen Überschüssen und/oder nicht umgesetzten Teilen der zur organischen Oberflächenmodifikation der Nanofüllstoffe verwendeten Verbindung umgesetzt wird.

Eine besonders bevorzugte Variante ist die Verwendung eines Mikrofüllstoffs, der röntgenopak ist und in solchen Mengen in das erfindungsgemäße Material eingearbeitet wird, dass das erfindungsgemäße Material eine Röntgenopazität (gemäß ISO 4049-2000) bevorzugt ≥ 100 % A1 und besonders bevorzugt ≥ 200 % A1 aufweist.

Das erfindungsgemäße polymerisierbare Material kann als ein lagerfähiges Einkomponentensystem ausgebildet sein, das beispielsweise durch einen Fotoinitiator und Licht zur Aushärtung gebracht wird. Bevorzugt ist im Rahmen der Erfindung jedoch die Ausbildung als ein Mehrkomponentensystem, insbesondere Zweikomponentensystem. Dementsprechend ist Gegenstand der Erfindung auch ein Kit zur Herstellung eines erfindungsgemäßen polymerisierbaren Materials, der wenigstens zwei Komponenten enthält, aus denen das polymerisierbare Material anmischbar ist.

Bei einem erfindungsgemäßen Kit können die Füllstoffe in beiden Komponenten enthalten sein. Alternativ ist es möglich, den Nanofüllstoff nur in eine Komponente des Kits einzubringen, der Mikrofüllstoff ist bevorzugt in beiden Komponenten des Kits enthalten.

Ein solcher Zweikomponentenkit kann entweder per Hand oder automatisch angemischt werden. Bei der automatischen Anmischung werden die beiden Komponenten in einer Doppelkammer-Kartusche gelagert und durch Herauspressen durch eine auf diese Kartusche aufgesetzte Mischkanüle homogen miteinander vermischt. Das Herauspressen der Komponenten kann entweder manuell oder mit Hilfe eines geeigneten Austraggerätes erfolgen, das in aller Regel eine höhere Auspresskraft erlaubt. Automatische Anmischung ist bevorzugt.

Um diese automatische Anmischbarkeit zu gewährleisten, ist es im Rahmen der Erfindung bevorzugt, dass die dynamische Viskosität jeder Komponente bei einer Schubspannung von 1.500 Pa zwischen 5 und 250 Pas liegt. Die Messung der dynamischen Viskosität erfolgt nach dem weiter unten im Zusammenhang mit den Beispielen beschriebenen Verfahren.

Bevorzugte Untergrenzen für die dynamische Viskosität bei einer Schubspannung von 1.500 Pa sind 8 und 10 Pas. Bevorzugte Obergrenzen sind 225, 200 und 175 Pas. Diese Unter-und Obergrenzen sind beliebig zu erfindungsgemäßen Bereichen kombinierbar.

Das erfindungsgemäße Material kann zur Einstellung bestimmter Eigenschaften zusätzlich weitere Bestandteile, u.a. auch sogenannte Additive bzw. Modifikatoren enthalten. Die Allgemeinheit nicht einschränkend sind nachfolgend einige Beispiele genannt: Gefüllte und/oder ungefüllte Splitterpolymerisate, gefüllte und/oder ungefüllte Perlpolymerisate. Diese Füllstoffbestandteile unterfallen den in Anspruch 1 verwendeten Begriffen "Mikrofüllstoff" und zählen zum Gesamtfüllstoffgehalt, wenn ihre mittlere Teilchengröße in den für Mikrofüllstoffe definierten Bereichen liegt. Liegt die mittlere Teilchengröße oberhalb diese Bereichs, zählen diese Füllstoffe zu den zusätzlichen Bestandteilen und nicht zum Gesamtfüllstoffgehalt. Solche zusätzlichen Bestandteile können auch pyrogene Kieselsäuren sein, deren mittlere Teilchengröße aufgrund der Agglomeration und/oder Aggregation von Primärpartikeln über der für Mikrofüllstoffe definierten Obergrenze liegt. Weitere mögliche zusätzliche Bestandteile sind anorganische und/oder organische Farbpigmente bzw. Farbstoffe, Stabilisatoren (wie z.B. substituierte und unsubstituierte Hydroxyaromaten, Tinuvine, Terpinene, Phenothiazin, sogenannte HALS - Hindered Amine Light Stabilizers - und/oder Schwermetallfänger wie EDTA), Weichmacher (wie z.B. Polethylenglykole, Polypropylenglykole, ungesättigte Polyester, Phthalate, Adipate, Sebacate, Phosphorsäureester, Phosphonsäureester und/oder zitronensäureester), ionenabgebende Substanzen, insbesondere solche die Fluoridionen freisetzen (wie z.B. Natriumfluorid, Kaliumfluorid und/oder quartäre Ammoniumfluoride), Röntgenkontrastmittel (wie z.B. Yttriumfluorid, Ytterbiumfluorid, Bismuthoxidchlorid und Bismuthtrifluorid) und/oder bakterizide bzw. antibiotisch wirksame Substanzen (wie z.B. Chlorhexidin, Pyridiniumsalze, Penicilline, Tetracycline, Chloramphenicol, antibakterielle Makrolide und/oder Polypeptid-Antibiotika).

Weichmacher (nicht reaktive organische Harzbestandteile) sind bevorzugt in einem Anteil von 8 Gew.-% oder weniger enthalten. Weitere bevorzugte Obergrenzen für den Anteil von Weichmachern sind 7 und 5 Gew.-%. Weiter bevorzugt kann das erfindungsgemäße Material keinerlei Weichmacher enthalten.

Das erfindungsgemäße Material enthält bevorzugt ein geeignetes Initiatorsystem. Es umfasst ein oder mehrere Initiatoren und optional ein oder mehrere Coinitiatoren.

Wie vorstehend bereits erwähnt, ist das erfindungsgemäße Material bevorzugt durch radikalische Polymerisation aushärtbar.

Dabei können Initiator bzw. Initiatoren und Coinitiator bzw. Coinitiatoren zusammen in einer Komponente und/oder getrennt in zwei oder mehreren Komponenten enthalten sein. Das erfindungsgemäße Material kann somit chemisch und/oder photochemisch, d.h. durch Bestrahlung mit UV- und/oder sichtbarem Licht, ausgehärtet werden. Dual aushärtende Materialien sind bevorzugt.

Die hier verwendbaren Initiatoren können z.B. Photoinitiatoren sein. Diese sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm und optional durch die zusätzliche Reaktion mit einem oder mehreren Coinitiatoren die Aushärtung des Materials bewirken können. Bevorzugt werden hier Phosphinoxide, Bezoinether, Benzilketale, Acetophenone, Benzophenone, Thioxanthone, Bisimidazole, Metallocene, Fluorone, α-Dicarbonylverbindungen, Aryldiazoniumsalze, Arylsulfoniumsalze, Aryliodoniumsalze, Ferroceniumsalze, Phenylphosphoniumsalze oder eine Mischung aus diesen Verbindungen eingesetzt.

Besonders bevorzugt werden Diphenyl-2,4,6-trimethylbenzoyl-phosphinoxid, Benzoin, Benzoinalkylether, Benzildialkylketale, α-Hydroxy-acetophenon, Dialkoxyacetophenone, α-Aminoacetophenone, i-Propylthioxanthon, Campherchinon, Phenylpropandion, 5,7-Diiodo-3-butoxy-6-fluoron, (eta-6-Cumol)(eta-5-cyclopentadienyl)eisen-hexafluorophosphat, (e-ta-6-Cumol)(eta-5-cyclopentadienyl)eisen-tetrafluoroborat, (eta-6-Cumol)(eta-5-cyclopentadienyl)eisen-hexafluoroantimonat, substituierte Diaryliodoniumsalze, Triarylsulfoniumsalze oder eine Mischung aus diesen Verbindungen eingesetzt.

Als Coinitiatoren für eine photochemische Aushärtung werden bevorzugt tertiäre Amine, Borate, organische Phosphite, Diaryliodoniumverbindungen, Thioxanthone, Xanthene, Fluorene, Fluorone, α-Dicarbonylverbindungen, kondensierte Polyaromaten oder eine Mischung aus diesen Verbindungen eingesetzt. Besonders bevorzugt werden N,N-Dimethyl-p-toluidin, N,N-Dialkyl-alkyl-aniline, N,N-Dihydroxyethyl-p-toluidin, elektronenarme tertiäre Amine wie z.B. 2-Ethylhexyl-p-(dimethyl-amino)-benzoat, Butyrylcholin-triphenylbutylborat oder eine Mischung aus diesen Verbindungen eingesetzt.

Für eine chemische Aushärtung bei Raum- bzw. Mundtemperatur wird i.a. ein Redoxinitiatorsystem verwendet, das aus einem bzw. mehreren Initiatoren und einem als Aktivator dienenden Coinitiator bzw. Coinitiatoren besteht. Aus Gründen der Lagerstabilität werden Initiator bzw. Initiatoren und Coinitiator bzw. Coinitiatoren in räumlich voneinander getrennten Teilen des erfindungsgemäßen Dentalmaterials eingearbeitet, d.h. es liegt ein mehrkomponentiges, bevorzugt ein zweikomponentiges Material vor. Als Initiator bzw. Initiatoren werden bevorzugt anorganische und/oder organische Peroxide, anorganische und/oder organische Hydroperoxide, Barbitursäurederivate, Malonylsulfamide, Protonensäuren, Lewis- oder Broensted-Säuren bzw. Verbindungen, die solche Säuren freisetzen, Carbeniumionen-Donatoren wie z.B. Methyltriflat oder Triethylperchlorat oder eine Mischung aus diesen Verbindungen und als Coinitiator bzw. als Coinitiatoren bevorzugt tertiäre Amine, Schwermetallverbindungen, insbesondere Verbindungen der 8. und der 9. Gruppe des Periodensystems ("Eisen- und Kupfergruppe"), Verbindungen mit ionogen gebundenen Halogenen oder Pseudohalogenen wie z.B. quartäre Ammoniumhalogenide, schwache Broenstedt-Säuren wie z.B. Alkohole und Wasser oder eine Mischung aus diesen Verbindungen eingesetzt.

In dem erfindungsgemäßen Dentalmaterial kann auch jede denkbare Kombination der oben beschriebenen Initiatoren und Coinitiatoren enthalten sein. Ein Beispiel hierfür sind sogenannte dualhärtende Dentalmaterialien, die sowohl Photoinitiatoren und optional die entsprechenden Coinitiatoren für eine photochemische Aushärtung als auch Initiatoren und entsprechende Coinitiatoren für eine chemische Aushärtung bei Raum- bzw. Mundtemperatur enthalten.

Die Erfindung wird nachfolgend anhand von Beispielen beschrieben. Zunächst werden zwei im Rahmen der Beispiele verwendete Messverfahren erläutert.

### 1. Messung der dynamischen viskosität

Wie vorstehend bereits erläutert, werden beim automatischen Anmischen von Zweikomponentensystemen die Komponenten aus den jeweiligen Kammern einer Kartusche herausgepresst und in einer Mischkanüle homogen miteinander vermischt. Um die Austragung des Materials aus den Kartuschen zu gewährleisten, darf die dynamische Viskosität unter den beim Auspressen vorherrschenden Schubspannungen nicht zu groß sein. Das Ausbringen der Komponenten erfolgt mit einer Krafteinwirkung und somit unter einer Schubspannung, die vom verwendeten Austraggerät abhängt. Bei der Verwendung eines kommerziellen Austraggerätes können Schubspannungen (τ) von bis zu etwa 500 bis 1500 Pa auftreten.

Ein Maß für die Fließfähigkeit einer Substanz ist ihre dynamische Viskosität (η), die sich aus dem Quotienten Schubspannung (τ) zu Schergeschwindigkeit (ý) ergibt: η = τ/ý [Pas].

Die dynamische Viskosität der einzelnen Komponenten der aufgeführten Beispiele wurde mit Hilfe eines Dynamic Stress Rheometers (DSR) der Firma Rheometrics bestimmt.

Alle Proben wurden zwischen 2 parallelen Platten mit einem Durchmesser von 25mm und einer Spaltweite von 0,5mm vermessen, wobei eine Temperatur von 23°C eingestellt wurde.

Bei jeder Messung wurde eine geeignete Probenmenge auf die untere, fest montierte Platte aufgebracht und dann wurde die Probe für 60s mit einer Schubspannung von 600Pa vorgeschert. Nach einer Ruhezeit von 30s wurde die Messung mit dem Messprogramm "Dynamischer Schubspannungssweep" des DSR gestartet. Dabei oszillierte die obere Platte mit einer Frequenz von 1Hz und bei vorgegebenen, schrittweise in Schritten von 10Pa ansteigenden Schubspannungen (Haltezeit jeweils 5s) von 10Pa bis 1500Pa wurde die dynamische Viskosität gemessen.

### 2. Messung der Standfestigkeit

Es ist von Vorteil, wenn das angemischte Stumpfaufbaumaterial vor dem Aushärten eine gewisse Standfestigkeit, d.h. eine ausreichend hohe Fließgrenze aufweist. Eine geringe Standfestigkeit kann bei bestimmten Anwendungen (beispielsweise Anwendungen im Oberkiefer) dazu führen, dass das Material auch im Ruhezustand bzw. bei geringen Schubspannungen oder Scherungen relativ stark fließt und damit den Stumpfaufbau erschwert. Zur Messung der Standfestigkeit kann der nachfolgend beschriebene Test durchgeführt werden.

Dieser Test leitet sich von einem Prüfverfahren der CRA (Clinical Research Associates) ab, mit dem die Viskosität bzw. die Standfestigkeit von Sealants qualitativ erfasst wurde (s. CRA Newsletter, August 2001).

Im oberen Bereich einer Glasplatte wurden jeweils 230mg des automatisch angemischten Stumpfaufbaumaterials aufgebracht. Sofort danach wurde die Glasplatte für 30s vertikal gehalten, wodurch die Materialien unterschiedlich stark nach unten flossen. Anschließend wurde die Glasplatte wieder horizontal gestellt und der komplette Materialstrang mit Hilfe einer LED-Handlampe (Typ Mini-LED der Firma Acteon) ausgehärtet.

Anschließend wird mit einer Schieblehre die Länge des ausgehärteten Materialstrangs gemessen. Im Rahmen der Erfindung ist es bevorzugt, dass die Stranglänge nicht größer als 55 mm ist. Weiter bevorzugt ist eine Obergrenze von 25 mm für die Stranglänge.

### 3. Teilchengrößenbestimmung der Nanofüllstoffe

Die Teilchengrößenbestimmungen wurden mittels dynamischer Lichtstreuung (3D-PCS) durchgeführt. Die Methode erlaubt die Bestimmung der Gewichtsanteile von Teilchengrößen im Bereich von 1 nm bis zu einigen Mikrometern. Die obere Grenze der Methode ist dadurch gegeben, dass größere Teilchen in der Messlösung sedimentieren und somit nicht erfasst werden können.

Alle Proben wurden als verdünnte Dispersionen in 2-Butanon untersucht, wobei ein Feststoffanteil von etwa 0,5 Gew-% eingestellt wurde. Diese verdünnung wurde in erster Linie gewählt, um Teilchen-Teilchen-Wechselwirkungen sicher auszuschließen.

### 4.Teilchengrößestimmung der Mikrofüllstoffe

Die Partikelgrößenverteilung wird mittels der Laserdiffraktometrie (Typ: Coulter LS 130) erfasst. Es wird bei diesem Verfahren der Gewichtsanteil von Partikeln, die eine bestimmte Größe haben, bestimmt. Ein Charakteristikum ist der d₅₀-Wert, der angibt, dass die Hälfte (50 %) der Gesamtmasse der Teilchen diese Größe über- bzw. unterschreiten. Die Vermessung der Teilchen erfolgt in verdünnten, zumeist wässrigen Dispersionen.

### 5. Druckfestigkeit

Für die Bestimmung der Druckfestigkeit wurden von jeder Probe 11-12 Probekörper vermessen. Zur Herstellung der Probekörper wurde eine aus rostfreiem Stahl bestehende Probekörperform, die eine 4mm hohe (+/-0,02) und 2mm weite (+/- 0,01) zylindrische Bohrung enthält, auf einer Polyethylenfolie platziert, die wiederum auf einem Objektträger aus Glas platziert war. Die Probe wurde blasenfrei in die Bohrung gegeben, wiederum mit der Folie und anschließend mit einem weiteren Objektträger abgedeckt. Überschüssige Probe wurde herausgepresst und die Objektträger mit einer Klemme fixiert. Die Proben wurden von beiden Seiten 40 s mit einer Polymerisationslampe (Espe Elipar II, 3m Espe AG, Deutschland) belichtet. Die erhärteten Probekörper wurden nass geschliffen (Schleifpapier, Körnung P 600) und anschließend aus der Probekörperform entnommen. Die Probekörper wurden 24 h bei 37°C in destilliertem Wasser gelagert.

Die Druckfestigkeit der Probekörper wurde mit einem Kraftmessgerät (Zwick Z010, Zwick GmbH & Co. KG, Deutschland) mit einer 10 kN-Messzelle bestimmt. Dabei wurde eine Prüfeinrichtung bestehend aus zwei parallelen Platten, wobei eine fest montiert war und die zweite mit der Kraftmesszelle verbunden war, verwendet. Die einzelnen Probekörper wurden aufrecht in die Mitte der fest montierten Platte gestellt und die zweite Platte wurde dann mit einer konstanten Vorschubgeschwindigkeit von 1,0 mm/min heruntergefahren, so dass die Belastung der Prüfkörper in Richtung ihrer Längsachse erfolgte.

### Beispiele 1 und 2

Bei diesen Beispielen handelt es sich um Zweikomponentensysteme, die beiden Komponenten werden als Basispaste (ATL-Paste) und Katalysatorpaste (K-Paste) bezeichnet. Die Pasten weisen die Zusammensetzung (Gewichtsangaben in g) gemäß der nachfolgenden Tabelle auf:

**Tabelle 1**

| **Beispiel 1** | | **Beispiel 2** | |
|---|---|---|---|
| **ATL-Paste** | | **ATL-Paste** | |
| Bis-GMA | 50,000 | Bis-GMA | 50,000 |
| TEDMA | 50,000 | TEDMA | 50,000 |
| Dimethyl-p-toluidin | 1,433 | Dimethyl-p-toluidin | 1,433 |
| | | | |
| Dimethylamino-benzoesäure-ethylester | 1,146 | Dimethylaminobenzoesäure-ethylester | 1,146 |
| Campherchinon | 0,573 | Campherchinon | 0,573 |
| BHT | 0,015 | BHT | 0,015 |
| Aerosil R 974 | 11,463 | Aerosil R 974 | 8,843 |
| Glaspulver silanisiert d50=2,5µm | 199,725 | Glaspulver sil. d50=1,5µm | 159,175 |
| Nanofüllstoff | 67,747 | Nanofüllstoff | 23,581 |
| | | | |

| **K-Paste (Katalysatorpas-te** | | **K-Paste** | |
|---|---|---|---|
| | | | |
| Bis-GMA | 50,000 | Bis-GMA | 50,000 |
| TEDMA | 50,000 | TEDMA | 50,000 |
| BPO | 0,839 | BPO | 0,839 |
| BHT | 0,226 | BHT | 0,226 |
| Methacrylsäure | 0,470 | Methacrylsäure | 0,470 |
| Aerosil R 974 | 11,282 | Aerosil R 974 | 8,703 |
| Glaspulver silanisiert d50=2,5µm | 196,563 | Glaspulver sil. d50=1,5µm | 156,653 |
| Nanofüllstoff | 66,674 | Nanofüllstoff | 23,208 |

### Nanofüllstoff:

280g Aerosil® 200 (Degussa AG) wurden in einen 4L-Zweihalskolben eingewogen und mit ca. 2000g 2-Butanon versetzt. Die zunächst breiige Masse wurde solange mit einem KPG-Rührer gerührt, bis sich eine homogene flüssige Suspension bildete. Dann wurden 178,36g 3-Methacryloxypropyltrimethoxysilan mit einer Dosierpumpe zugetropft. Die dünnflüssige Suspension wurde insgesamt 48 Stunden bei Raumtemperatur gerührt. Anschließend wurde das 2-Butanon langsam an einem Rotationsverdampfer abgezogen. Nach dem Entfernen des Lösungsmittels blieb ein weißes, lockeres, grobteiliges poröses Pulver zurück, das leicht zerfiel.

### Nanodispersion (Vorstufe der Erfindungsbeispiele):

240g einer 1:1-Mischung aus Bisphenol-A-diglycidylmethacrylat (Bis-GMA) und Triethylenglykoldimethacrylat (TEDMA) wurden in einen 1000ml Dispergiertopf gegeben. Dann wurden 160g des Nanofüllstoffs in Portionen von ca. 20-40g mit Hilfe eines Dispermaten (Typ AE04-C1 der Firma VMA-Getzmann), unter Verwendung einer Dissolverscheibe mit einem Durchmesser von 70mm, in ca. 60 Minuten bei Umdrehungsgeschwindigkeiten von 250 U/min bis 1250 U/min eingearbeitet. Anschließend wurde für ca. 120 Minuten bei 2000 U/min weiter dispergiert. Es entstand eine nahezu farblose und transparente Nanodispersion.

### Beispiel 1:

Es wurden eine Basispaste (ATL-Paste) und eine Katalysator-Paste (K-Paste) hergestellt.

### 1) ATL-Paste:

In 132,975g der Nanodispersion wurden 1,125g N,N-Dimethyl-p-toluidin, 0,9g Dimethylaminobenzoesäure-ethylester, 0,45g Campherchinon und 0,012g 3,5-Bis-tert.-butyl-4-hydroxytoluol (BHT) durch Rühren gelöst. Dann wurden 9,0g Aerosil R974 (Degussa AG) und 156,81g mit 3-Methacryloxypropyltrimethoxysilan silanisiertes BariumSilikatglas (Typ GM27884 K6 der Firma Schott AG) mit Hilfe eines Planetenmischers (Typ LPV 0.3-1 der Firma PC Laborsystem) homogen eingearbeitet. Die Paste wurde zweimal unter Verwendung eines Labor-Dreiwalzenstuhls (Typ 50 der Firma Exakt) gewalzt (Spalt-Einstellung: 2/2) und anschließend unter Rühren für 30min bei ca. 200mbar entgast.

### 2) K-Paste:

In 132,975g der Nanodispersion wurden 0,669g Dibenzoylperoxid, 0,375g Methacrylsäure und 0,18g 3,5-Bis-tert.-butyl-4-hydroxytoluol (BHT) durch Rühren gelöst. Dann wurden 9,0g Aerosil R974 (Degussa AG) und 156,81g mit 3-Methacryloxypropyltrimethoxy-silan silanisiertes BariumSilikatglas (Typ GM27884 K6 der Firma Schott AG) mit Hilfe eines Planetenmischers (Typ LPV 0.3-1 der Firma PC Laborsystem) homogen eingearbeitet. Die Paste wurde zweimal unter Verwendung eines Labor-Dreiwalzenstuhls (Typ 50 der Firma Exakt) gewalzt (Spalt-Einstellung: 2/2) und anschließend unter Rühren für 30min bei ca. 200mbar entgast.

### Beispiel 2:

Es wurden eine Basispaste (ATL-Paste) und eine Katalysator-Paste (K-Paste) hergestellt.

### 1) ATL-Paste:

60g der Nanodispersion wurden mit 65,776g einer 1:1-Mischung aus Bisphenol-A-diglycidylmethacrylat (Bis-GMA) und Triethylenglykoldimethacrylat (TEDMA) homogen vermischt. In dieser Mischung wurden 1,458g N,N-Dimethyl-p-toluidin, 1,167g Dimethylaminobenzoesäure-ethylester, 0,583g Campherchinon und 0,016g 3,5-Bis-tert.-butyl-4-hydroxytoluol (BHT) durch Rühren gelöst. Dann wurden 9,0g Aerosil R974 (Degussa AG) und 162,0g mit 3-Methacryloxypropyltrimethoxy-silan silanisiertes BariumSilikatglas (Typ GM 27884 UF1.5 der Firma Schott AG) mit Hilfe eines Planetenmischers (Typ LPV 0.3-1 der Firma PC Laborsystem) homogen eingearbeitet. Die Paste wurde zweimal unter Verwendung eines Labor-Dreiwalzenstuhls (Typ 50 der Firma Exakt) gewalzt (Spalt-Einstellung: 2/2) und anschließend unter Rühren für 30min bei ca. 200mbar entgast.

### 2) K-Paste:

60g der Nanodispersion wurden mit 67,413g einer 1:1-Mischung aus Bisphenol-A-diglycidylmethacrylat (Bis-GMA) und Triethylenglykoldimethacrylat (TEDMA) homogen vermischt. In dieser Mischung wurden 0,867g Dibenzoylperoxid, 0,486g Methacrylsäure und 0,233g 3,5-Bis-tert.-butyl-4-hydroxytoluol (BHT) durch Rühren gelöst. Dann wurden 9,0g Aerosil R974 (Degussa AG) und 162,0g mit 3-Methacryloxypropyltrimethoxy-silan silanisiertes BariumSilikatglas (Typ GM27884 UF1.5 der Firma Schott AG) mit Hilfe eines Planetenmischers (Typ LPV 0.3-1 der Firma PC Laborsystem) homogen eingearbeitet. Die Paste wurde zweimal unter Verwendung eines Labor-Dreiwalzenstuhls (Typ 50 der Firma Exakt) gewalzt (Spalt-Einstellung: 2/2) und anschließend unter Rühren für 30min bei ca. 200mbar entgast.

### Vergleichsbeispiele 1 und 2

Als Vergleichsbeispiele 1 und 2 wurden die Beispiele 2 und 3 aus dem Stand der Technik gemäß EP 1 790 323 A1 nachgestellt. Deren Zusammensetzung ist beschrieben auf Seite 17 dieses Standes der Technik in der Tabelle 1 als Embodiment 2 und Embodiment 3. Embodiment 2 entspricht dem vorliegenden Vergleichsbeispiel 1, Embodiment 3 dem vorliegenden Vergleichsbeispiel 2.

### Beispiel 3

### Ermittlung der dynamischen Viskositäten

Die bei Schubspannungen von 500 bis 1500 Pa ermittelten dynamischen viskositäten der Komponenten der Beispiele sind in der nachfolgenden Tabelle 2 zusammengefasst.

| | **Basis-Paste (ATL)** | | | **Katalysator-Paste (K)** | | |
|---|---|---|---|---|---|---|
| | **DSR-Viskosität η [Pas]** | | | **DSR-Viskosität η [Pas]** | | |
| | **τ=500Pa** | **τ=1000Pa** | **τ=1500Pa** | **τ=500Pa** | **τ=1000Pa** | **τ=1500Pa** |
| **Vgl.-Bsp. 1** | 38 | 46 | 51 | 85 | 105 | 106 |
| **vgl.-Bsp. 2** | 30 | 23 | 17 | 16 | 17 | 17 |
| **Bsp. 1** | 101 | 130 | 136 | 165 | 217 | 239 |
| **Bsp. 2** | 11 | 12 | 13 | 15 | 15 | 15 |

Alle Pastenkombination konnten mit einem sensorisch akzeptablen Kraftaufwand ausgebracht werden.

### Beispiel 4

### Messung der Beschleifbarkeit

Für die Beispiele und Vergleichsbeispiele wird die Beschleifbarkeit im vergleich zu Humandentin nach dem oben beschriebenen Verfahren gemessen. Von jedem Beispiel bzw. Vergleichsbeispiel werden vier Prüfkörper hergestellt, die angegebene Zeit ist der Mittelwert aus vier Messungen.

Alle aufgeführten Beispiele und Vergleichsbeispiele wurden in schwarze 25ml 1:1-Doppelkammer-Kartuschen (Teil-Nr.: CS 025-01-13) der Firma Mixpac Systems AG abgefüllt und gelagert. Bei der Herstellung der Prüfkörper für die nachfolgenden Messungen von Beschleifbarkeit und Druckfestigkeit wurden beide Komponenten automatisch angemischt. Dabei wurden jeweils die gelbe Mischkanüle MB 4.2-12-D (Mixpac Systems AG) und das Austraggerät DS 24-01-00 (ebenfalls Mixpac Systems AG) verwendet.

Die Messergebnisse sind in der nachfolgenden Tabelle 3 angegeben.

**Tabelle 3**

| | Zeit [s] | Standardabweichungen [s] |
|---|---|---|
| Humandentin | 9,56 | 1,48 |
| Beispiel 1 | 9,14 | 1,46 |
| Beispiel 2 | 9,94 | 1,43 |
| Vergleichsbeispiel 1 | 6,47 | 0,54 |
| Vergleichsbeispiel 2 | 5,89 | 1,23 |

Man erkennt, dass die Beschleifbarkeit der erfindungsgemäßen Beispiele derjenigen von Humandentin sehr ähnlich ist, während die Beschleifbarkeit der Vergleichsbeispiele des Standes der Technik deutlich abweicht.

Die Beschleifbarkeitsmessungen wurden mit dem vorstehend angegebenen Diamantschleifkörper der Firma Brasseler durchgeführt. Es sei angemerkt, dass im Rahmen der Erfindung für die Messungen andere vergleichbare Diamantschleifkörper (vorzugsweise mit einer Körnung von 100 µm) verwendet werden können. Da Patentanspruch 1 der Erfindung die Beschleifbarkeit eines erfindungsgemäßen Stumpfaufbaumaterials relativ zu der Beschleifbarkeit von Humandentin definiert, kommt es lediglich darauf an, dass Messung und Vergleichsmessung jeweils unter den gleichen Bedingungen mit dem gleichen Schleifkörper durchgeführt werden.

### Beispiel 5

### Messung der Druckfestigkeit

Die Druckfestigkeit der Prüfkörper gemäß den Beispielen und Vergleichsbeispielen wurde nach dem oben beschriebenen Verfahren gemessen.

**Tabelle 4**

| | Druckfestigkeit [MPa] |
|---|---|
| Beispiel 1 | 404 ± 33 |
| Beispiel 2 | 447 ± 26 |
| Vergleichsbeispiel 1 | 323 ± 18 |
| Vergleichsbeispiel 2 | 298 ± 20 |

Man erkennt, dass die Druckfestigkeit der erfindungsgemäßen Stumpfaufbaumaterialien deutlich höher ist als im Stand der Technik.

## Patentansprüche

1. Polymerisierbares Material mit den Merkmalen:
- das Material enthält wenigstens ein polymerisierbares Monomer, Oligomer und/oder Präpolymer,
- der Gesamtfüllstoffgehalt des Materials beträgt 50 bis 80 Gew.-%,
- das Material enthält 6 bis 18 Gew.-% Nanofüllstoff mit einer mittleren Teilchengröße, bestimmt mittels dynamischer Lichtstreuung (3D-PCS) an verdünnten Dispersionen in 2-Butanon mit einem Feststoffanteil von etwa 0,5 Gew-%, d₅₀ von 300 nm oder weniger, wobei wenigstens 50 Gew.-% dieses Nanofüllstoffs eine Teilchengröße von 200 nm oder weniger aufweist,
- das Material enthält 35 bis 60 Gew.-% Mikrofüllstoff mit einer mittleren Teilchengröße, bestimmt mittels Laserdiffraktometrie, vom Typ Coulter LS130 d₅₀ von 0,5 bis 10 µm,
- die Beschleifbarkeit des ausgehärteten Stumpfaufbaumaterials weicht höchstens 15% von der Beschleifbarkeit von Humandentin ab, wobei diese Beschleifbarkeit wie folgt ermittelt wird:
- es wird ein zylindrischer Diamantschleifkörper mit einer Körnung von 100 µm (Typ ISO 836.314.012) bei 150.000 U/min verwendet,
- der Prüfkörper wird µm einen Betrag von 0,2 mm an den Schleifkörper herangeführt,
- der Prüfkörper wird mit einem Zuggewicht von 100 g an dem Diamantschleifkörper vorbeigeführt und beschliffen,
- gemäß ISO-Norm 11405 wird der Schleifkörper mit Spraywasser bei einer Durchflussgeschwindigkeit von 50 ml/min und einer Temperatur von 23°C gekühlt ,
- es wird die Zeit gemessen, die der Schleifkörper für das Zurücklegen einer Strecke von 10 mm auf der Prüfkörperoberfläche benötigt,
zur Verwendung als dentales Stumpfaufbaumaterial.

2. Material nach Anspruch 1, **dadurch gekennzeichnet, dass** die polymerisierbaren Monomere, Oligomere und/oder Präpolymere ausgewählt sind aus der Gruppe bestehend aus radikalisch und kationisch polymerisierbaren Monomeren, Oligomeren und/oder Präpolymeren, wobei vorzugsweise die polymerisierbaren Monomere Acrylate und/oder Methacrylate umfassen.

3. Material nach Anspruch 1 oder 2, **dadurch gekennzeichnet, dass** der Gesamtfüllstoffgehalt wenigstens 55 Gew.-%, vorzugsweise wenigstens 58 Gew.-%, weiter vorzugsweise wenigstens 60 Gew.-% beträgt, und/oder dass der Gesamtfüllstoffgehalt höchstens 75 Gew.-%, weiter vorzugsweise höchstens 72 Gew.-%, weiter vorzugsweise höchstens 70 Gew.-% beträgt.

4. Material nach einem der Ansprüche 1 bis 3, **dadurch gekennzeichnet, dass** der Gehalt an Nanofüllstoff wenigstens 8 Gew.-% beträgt .

5. Material nach einem der Ansprüche 1 bis 4, **dadurch gekennzeichnet, dass** wenigstens 50 Gew.-% des Nanofüllstoffs eine Teilchengröße von 150 nm oder weniger, bevorzugt 100 nm oder weniger aufweist.

6. Material nach einem der Ansprüche 1 bis 5, **dadurch gekennzeichnet, dass** die mittlere Primärteilchengröße des Nanofüllstoffs, bestimmt mittels Transmissions-elektronenmikroskopie, zwischen 1 und 80 nm, vorzugsweise 4 und 60 nm, weiter vorzugsweise 6 und 50 nm liegt.

7. Material nach einem der Ansprüche 1 bis 6, **dadurch gekennzeichnet, dass** der Gehalt an Mikrofüllstoff wenigstens 40 Gew.-%, vorzugsweise wenigstens 45 Gew.-% beträgt.

8. Material nach einem der Ansprüche 1 bis 7, **dadurch gekennzeichnet, dass** die mittlere Teilchengröße d₅₀ des Mikrofüllstoffs wenigstens 0,7 µm, weiter vorzugsweise wenigstens 1,0 µm beträgt, und/oder dass die mittlere Teilchengröße d₅₀ des Mikrofüllstoffs höchstens 7,0 µm, vorzugsweise höchstens 5,0 µm, weiter vorzugsweise höchstens 4,0 µm, weiter vorzugsweise höchstens 3,0 µm beträgt.

9. Material nach einem der Ansprüche 1 bis 8, **dadurch gekennzeichnet, dass** der Nanofüllstoff und/oder der Mikrofüllstoff wenigstens teilweise oberflächenmodifiziert ist und funktionelle Gruppen auf seiner Oberfläche aufweist, die mit der organischen Matrix des Materials chemisch reagieren können oder eine hohe Affinität zu dieser Matrix haben.

10. Kit zur Herstellung eines polymerisierbaren Materials wie definiert in einem der Ansprüche 1 bis 9, **dadurch gekennzeichnet, dass** der Kit wenigstens zwei Komponenten enthält, aus denen das polymerisierbare Material anmischbar ist.

11. Kit nach Anspruch 10, **dadurch gekennzeichnet, dass** der Nanofüllstoff nur in einer Komponente enthalten ist.

12. Kit nach Anspruch 10 oder 11, **dadurch gekennzeichnet, dass** die dynamische Viskosität jeder Komponente, gemessen zwischen 2 parallelen Platten mit einem Durchmesser von 25mm und einer Spaltweite von 0,5mm bei einer Temperatur von 23°C, bei einer Schubspannung von 1.500 Pa zwischen 5 und 250 Pas liegt.

13. Kit nach Anspruch 12, **dadurch gekennzeichnet, dass** die dynamische Viskosität jeder Komponente bei einer Schubspannung von 1.500 Pa wenigstens 8 Pas, vorzugsweise wenigstens 10 Pas beträgt.

14. Kit nach Anspruch 12 oder 13, **dadurch gekennzeichnet, dass** die dynamische Viskosität jeder Komponente bei einer Schubspannung von 1.500 Pa höchstens 225 Pas, vorzugsweise höchstens 200 Pas, weiter vorzugsweise höchstens 175 Pas beträgt.

## Claims

1. A polymerisable material having the features:
- the material contains at least one polymerisable monomer, oligomer and/or prepolymer,
- the total filler content of the material amounts to 50 to 80 wt.%,
- the material contains 6 to 18 wt.% of nanofiller with an average particle size, determined by dynamic light scattering (3D-PCS) on dilute dispersions in 2-butanone with a solids content of approx. 0.5 wt.%, d₅₀ of 300 nm or less, at least 50 wt.% of this nanofiller having a particle size of 200 nm or less,
- the material contains 35 to 60 wt.% of microfiller with an average particle size, determined by Coulter LS130 laser diffractometry, d₅₀ of 0.5 to 10 µm,
- the grindability of the cured core build-up material deviates by at most 15% from the grindability of human dentine, said grindability being determined as follows:
- a cylindrical diamond burr with a grain size of 100 µm (type ISO 836.314.012) is used at 150,000 rpm,
- the test specimen is advanced towards the burr by an amount of 0.2 mm,
- the test specimen is conveyed past the diamond burr with a tractive weight of 100 g and ground,
- according to ISO standard 11405, the burr is cooled with a water spray at a flow rate of 50 ml/min and a temperature of 23°C,
- the time which it takes for the burr to cover a distance of 10 mm on the surface of the test specimen is measured,
for use as a dental core build-up material.

2. A material according to claim 1, **characterised in that** the polymerisable monomers, oligomers and/or prepolymers are selected from the group consisting of free-radically and cationically polymerisable monomers, oligomers and/or prepolymers, the polymerisable monomers preferably comprising acrylates and/or methacrylates.

3. A material according to claim 1 or claim 2, **characterised in that** the total filler content amounts to at least 55 wt.%, preferably at least 58 wt.%, more preferably at least 60 wt.%, and/or **in that** the total filler content amounts to at most 75 wt.%, more preferably at most 72 wt.%, more preferably at most 70 wt.%.

4. A material according to any one of claims 1 to 3, **characterised in that** the content of nanofiller amounts to at least 8 wt.%.

5. A material according to any one of claims 1 to 4, **characterised in that** at least 50 wt.% of the nanofiller has a particle size of 150 nm or less, preferably of 100 nm or less.

6. A material according to any one of claims 1 to 5, **characterised in that** the average primary particle size of the nanofiller, determined by transmission electron microscopy, is between 1 and 80 nm, preferably 4 and 60 nm, more preferably 6 and 50 nm.

7. A material according to any one of claims 1 to 6, **characterised in that** the content of microfiller amounts to at least 40 wt.%, preferably at least 45 wt.%.

8. A material according to any one of claims 1 to 7, **characterised in that** the average particle size d₅₀ of the microfiller amounts to at least 0.7 µm, more preferably at least 1.0 µm, and/or **in that** the average particle size d₅₀ of the microfiller amounts to at most 7.0 µm, preferably at most 5.0 µm, more preferably at most 4.0 µm, more preferably at most 3.0 µm.

9. A material according to any one of claims 1 to 8, **characterised in that** the nanofiller and/or the microfiller is/are at least in part surface-modified and comprise(s) functional groups on the surface thereof which are capable of reacting chemically with the organic matrix of the material or have an elevated affinity for said matrix.

10. A kit for producing a polymerisable material as defined in any one of claims 1 to 9, **characterised in that** the kit contains at least two components from which the polymerisable material may be mixed.

11. A kit according to claim 10, **characterised in that** the nanofiller is present in just one component.

12. A kit according to claim 10 or claim 11, **characterised in that** the dynamic viscosity of each component, measured between 2 parallel plates with a diameter of 25 mm and a gap width of 0.5 mm at a temperature of 23°C and a shear stress of 1,500 Pa, is between 5 and 250 Pa.s.

13. A kit according to claim 12, **characterised in that** the dynamic viscosity of each component at a shear stress of 1,500 Pa amounts to at least 8 Pa.s, preferably at least 10 Pa.s.

14. A kit according to claim 12 or claim 13, **characterised in that** the dynamic viscosity of each component at a shear stress of 1,500 Pa amounts to at most 225 Pa.s, preferably at most 200 Pa.s, more preferably at most 175 Pa.s.

## Revendications

1. Matériau polymérisable, avec les caractéristique :
- le matériau contient au moins un monomère, un oligomère et/ou un prépolymère polymérisable,
- la teneur totale en charge du matériau est de 50 à 80 % en poids,
- le matériau contient 6 à 18 % en poids de charge nanoscopique avec une taille moyenne de particule d₅₀, déterminée par diffraction dynamique de lumière (3D-PCS) sur des dispersions diluées dans de la 2-butanone avec une teneur en solide d'environ 0,5 % en poids, de 300 nm ou moins, 50 % en poids de cette charge nanoscopique présentant une taille de particule de 200 nm ou moins,
- le matériau contient 35 é 60 % en poids de charge microscopique avec une taille moyenne de particule d₅₀, déterminée par diffractométrie au laser de type Coulter LS130, de 0,5 à 10 µm,
- l'aptitude au ponçage du matériau de construction sur moignon durci diffère au plus de 15 % de l'aptitude au ponçage de la dentine humaine, cette aptitude au ponçage étant déterminée comme suit :
- on utilise une meule cylindrique à diamant avec une granulométrie de 100 µm (type ISO 836.314.02 à 150.000 tr/min,
- l'échantillon est amené contre la meule moyennant une valeur absolue 0,2 mm,
- l'échantillon est déplacé avec un poids de traction de 100 g devant la meule à diamant et est poncé,
- conformément à la norme ISO 11405, la meule est refroidie par pulvérisation d'eau avec un débit de 50 ml/min et une température de 23 °C
- le temps que nécessite la meule pour parcourir une distance de 10 mm sur la surface de l'échantillon est mesuré,
destiné à une utilisation comme matériau dentaire de construction sur moignon.

2. Matériau selon la revendication 1, **caractérisé en ce que** les monomères, les oligomères et/ou les prépolymères polymérisables sont choisis parmi le groupe consistant en des monomères, des oligomères et/ou des prépolymères polymérisables radicalaire-ment et cationiquement, les monomères polymérisables comprenant de préférence des acrylates et/ou des méthacrylates.

3. Matériau selon la revendication 1 ou 2, **caractérisé en ce que** la teneur totale en charge est d'au moins 55 % en poids, de préférence d'au moins 58 % en poids, encore plus préférablement d'au moins 60 % en poids, et/ou **en ce que** la teneur totale en charge est d'au plus 75 % en poids, de préférence d'au plus 72 % en poids, encore plus préférablement d'au plus 70 % en poids.

4. Matériau selon l'une quelconque des revendications 1 à 3, **caractérisé en ce que** la teneur en nanocharge est d'au moins 8 % en poids.

5. Matériau selon l'une quelconque des revendications 1 à 4, **caractérisé en ce qu'**au moins 50 % en poids de la nanocharge présente une taille de particule de 150 nm ou moins, de préférence de 100 nm ou moins.

6. Matériau selon l'une quelconque des revendications 1 à 5, **caractérisé en ce que** la taille moyenne des particules primaires de la nanocharge, déterminée par microscopie électronique à transmission, est comprise entre 1 et 80 nm, de préférence entre 4 et 60 nm, encore plus préférablement entre 6 et 50 nm.

7. Matériau selon l'une quelconque des revendications 1 à 6, **caractérisé en ce que** la teneur en microcharge est d'au moins 40 % en poids, de préférence d'au moins 45 % en poids.

8. Matériau selon l'une quelconque des revendications 1 à 7, **caractérisé en ce que** la taille moyenne de particule d₅₀ de la charge microscopique est au moins de 0,7 µm, encore plus préférablement au moins de 1,0 µm, et/ou **en ce que** la taille moyenne de particule d₅₀ de la charge microscopique est au plus de 7,0 µm, de préférence au plus de 5,0 µm, encore plus préférablement au plus de 4,0 µm, de façon particulièrement préférée au plus de 3,0 µm.

9. Matériau selon l'une quelconque des revendications 1 à 8, **caractérisé en ce que** la nanocharge et/ou la microcharge sont au moins partiellement modifiées en surface et comportent à leurs surfaces des groupes fonctionnels qui peuvent réagir chimiquement avec la matrice organique du matériau ou qui ont une haute affinité pour cette matrice.

10. Kit pour la préparation d'un matériau polymérisable comme défini dans l'une quelconque des revendications 1 à 9, **caractérisé en ce que** le kit contient au moins deux composants à partir desquels le matériau polymérisable peut être obtenu par mélangeage.

11. Kit selon la revendication 10, **caractérisé en ce que** la nanocharge n'est contenue que dans un composant.

12. Kit selon la revendication 10 ou 11, **caractérisé en ce que** la viscosité dynamique de chaque composant, mesurée entre 2 plaques parallèles d'un diamètre de 25 mm et d'une largeur de fente de 0,5 mm à une température de 23 °C, à une contrainte de cisaillement de 1.500 Pa, est comprise entre 5 et 250 Pa.s.

13. Kit selon la revendication 12, **caractérisé en ce que** la viscosité dynamique de chaque composant à une contrainte de cisaillement de 1.500 Pa, est d'au moins 8 Pa.s, de préférence d'au moins 10 Pa.s.

14. Kit selon la revendication 12 ou 13, **caractérisé en ce que** la viscosité dynamique de chaque composant à une contrainte de cisaillement de 1.500 Pa, est d'au plus 225 Pa.s, de préférence d'au plus 200 Pa.s, encore plus préférablement d'au plus 175 Pa.s.
